# EUROPÄISCHE PATENTSCHRIFT

(11) **EP 0 702 903 B1**
(45) Veröffentlichungstag und Bekanntmachung des Hinweises auf die Patenterteilung: **06.12.2000**
(21) Anmeldenummer: 95114669.5
(22) Anmeldetag: 18.09.1995
(51) Int. Cl.: A23L 1/32, A23L 1/015, C11B 1/10, A23J 7/00, C07F 9/10, B01D 11/02

(54) **Verfahren zur Herstellung von fett- und cholesterin-reduzierten pulverförmigen Produkten auf Eibasis mit einem hohen Gehalt an Phospholipiden**
Process for producing fat- and cholesterol-reduced egg-based powdery products of high phospholipid content
Procédé pour la préparation de produits pulvérulants à base d'oeufs avec une teneur réduite en graisse et en cholestérol, et à haute teneur en phospholipides

(30) Priorität: 19.09.1994 DE 4433274
(43) Veröffentlichungstag der Anmeldung: 27.03.1996
(73) Patentinhaber: SKW Trostberg Aktiengesellschaft, 83308 Trostberg (DE)
(72) Erfinder: Heidlas, Jürgen, Dr., D-83308 Trostberg (DE); Vollbrecht, Heinz-Rüdiger, Dr., D-83352 Altenmarkt (DE); Cully, Jan, Dr., D-84518 Garching (DE)
(74) Vertreter: Huber, Bernhard, Dipl.-Chem.

(56) Entgegenhaltungen:
- EP-A- 0 129 739
- EP-A- 0 352 667
- EP-A- 0 426 425
- WO-A-93/19617
- FOOD MARKETING AND TECHNOLOGY, Dezember 1994 Seiten 38-43, J.E. HEIDLAS 'PROPANE EXTRACTION IN FOOD PROCESSING'
- DATABASE WPI Week 8925 Derwent Publications Ltd., London, GB; AN 89-184023 & JP-A-01 123 864 (MORI SEIYU KK) , 16.Mai 1989 & PATENT ABSTRACTS OF JAPAN vol. 013 no. 367 (C-626) ,15.August 1989 & JP-A-01 123864 (MORI SEIYU KK) 16.Mai 1989,
- JAOCS, Bd. 62, Nr. 8, 1985 Seiten 1222-1230, R. EGGERS 'HIGH PRESSURE EXTRACTION OF OIL SEED'
- ANALYTICAL CHEMISTRY, Bd. 66, Nr. 5, 1994 Seiten 603-609, J.C. VIA ET AL 'SUPERCRITICAL FLUID FRACTIONATION OF A LOW MOLECULAR WEIGHT, HIGH-DENSITY POLYETHYLENE WAX USING CARBON DIOXIDE, PROPANE, AND PROPANE-MODIFIED CARBON DIOXIDE'

## Beschreibung

Die Erfindung betrifft ein Verfahren zur Herstellung von fett- und cholesterinreduzierten pulverförmigen Produkten auf Eibasis, die sich durch einen hohen Phospholipidgehalt auszeichnen.

Cholesterin und Cholesterinester (im folgenden als Cholesterinderivate bezeichnet) sind fettlösliche Verbindungen, die im Fettanteil tierischer Nahrungsmittel, wie z. B. Eigelb oder Fleisch, vorkommen.

Internationale Gesundheitsorganisationen empfehlen der Bevölkerung der hochentwickelten Länder, die Aufnahme zu hoher Fett- und Cholesterinmengen einzuschränken, da epidemologische Studien eindeutig eine ernährungsbedingte Ursache von Arteriosklerose und koronaren Herzkrankheiten nachweisen konnten.

Aufgrund dieser Empfehlungen ist die moderne Lebensmitteltechnologie gefordert, verbesserte und den Ernährungsgewohnheiten entsprechende Lebensmittel mit reduziertem Fett- und Cholesteringehalt den Verbrauchern anzubieten. Entscheidende Kriterien sind dabei, daß die organoleptischen, ernährungsphysiologischen und - aus lebensmitteltechnologischer Sicht - die funktionellen Eigenschaften der Produkte weitgehend erhalten bleiben.

Hinsichtlich der Cholesterinreduzierung sind bereits eine Reihe von Verfahren zur Entfernung von Cholesterinderivaten bekannt, doch eignen sich diese Verfahren wegen ihrer geringen Selektivität oder wegen der chemischen Veränderung wichtiger Bestandteile des Ausgangsmaterials (wie z. B. Denaturierung der Proteine) nicht oder nur wenig zur Reduktion des Cholesteringehalts in hochwertigen Lebensmitteln.

Im Gegensatz dazu ist die Hochdruckextraktion mit überkritischem C0₂ ein relativ schonendes Verfahren zur Entfernung von Fetten und Cholesterinderivaten (vgl. V. Krukonis, Supercritical Fluid Processing, International Symposium on Supercritical Fluids, Nice, 1988, und A. Bude und D. Knorr, Reduction of Cholesterol in Egg Powder and Whole Eggs by Extraction with Supercritical Carbon Dioxide, Fifth International Congress on Engineering and Food, 1989). Jedoch sind alle Verfahren, die mit überkritischem C0₂ arbeiten, mit den Nachteilen behaftet, daß sehr hohe Verfahrensdrücke (> 250 bar) notwendig sind, um eine entsprechende Gasbeladung zu erzielen und außerdem sehr hohe spezifische Gasdurchsatzraten benötigt werden, um das gewünschte Extraktionsziel, nämlich eine weitgehende Cholesterinreduzierung, zu erreichen. Damit wird die ökonomische Gesamtbilanz der C0₂-Verfahren in der Regel so verschlechtert, daß sie unwirtschaftlich werden kann. Die C0₂-Extraktion zur Entfernung von Cholesterinderivaten aus Eigelb wird bspw. in der EP-A 416 561 beschrieben.

Im Gegensatz zu C0₂ zeigt verdichtetes Propan in einem relativ niedrigen Druckbereich ein sehr gutes Lösevermögen für Fette und Öle, das verfahrenstechnisch seit längerer Zeit ausgenutzt wurde (vgl. bspw. US 2 560 935, US 4 331 695, DE-PS 23 63 418). Ein Nachteil der Propanextraktion ist die geringe Selektivität gegenüber den verschiedenen lipophilen Inhaltsstoffen, wie Triglyceriden, Cholesterinderivaten und Phospholipiden.

Es wurde auch schon vorgeschlagen, die Löslichkeit von Fetten/Ölen und oder fettähnlichen Stoffen in überkritischem CO₂ zu verbessern, indem diverse Schleppmittel, u. a. auch Propan, dem Extraktionsmedium (CO₂) zugespeist werden (vgl. G. Brunner, Stofftrennung mit überkritischen Gasen, Chem.-Ing.-Tech. 59 (1987), S. 12 - 22). Daraus kann aber der technologisch wichtige Aspekt nicht geklärt werden, ob die Selektivität der Extraktionsgase gezielt gesteuert werden kann, um die Extraktion bestimmter Komponenten, insbesondere Phospholipide, zu beeinflussen und wie die hierfür notwendigen Bedingungen festgestellt werden können.

Der Erfindung liegt daher die Aufgabe zugrunde, ein Verfahren zur Herstellung von fett- und cholesterinreduzierten pulverförmigen Produkten auf Eibasis mit verdichteten Gasen zu entwickeln, das die erwähnten Nachteile des Standes der Technik nicht aufweist, sondern es wirtschaftlich ermöglicht, die Menge der Fette und Cholesterinderivate in den Eiprodukten drastisch zu verringern, ohne hierbei größere Mengen der technologisch wichtigen Phospholipide, und insbesondere des Lecithins, zu entfernen.

Diese Aufgabe wird erfindungsgemäß dadurch gelöst, daß man das pulverförmige Ausgangsmaterial mit einer Mischung aus Propan und CO₂ in einem Mischungsverhältnis zwischen 95/5 und 5/95 Gew.-% bei einem Druck < 300 bar und einer Temperatur < 70°C extrahiert. Es hat sich nämlich überraschenderweise gezeigt, daß man bei der Einhaltung dieser Extraktionsbedingungen die selektive Entfernung der Fette und Cholesterinderivate erreicht, während die Extraktion der Phospholipide gezielt gesteuert und somit auch vollständig zurückgedrängt werden kann, so daß die funktionalen Eigenschaften des Eiproduktes vollständig erhalten bleiben.

Für das erfindungsgemäße Verfahren werden pulverförmige Produkte auf Eibasis, wie z. B. Eigelbpulver oder Volleipulver, oder Produkte, die diese Stoffe enthalten, eingesetzt. Es ist erfindungswesentlich, daß die Extraktion mit einer Mischung aus Propan und CO₂ in einem Mischungsverhältnis zwischen 95/5 und 5/95 Gew.-% bei einem Druck < 300 bar und einer Temperatur < 70°C durchgeführt wird. Auf diese Weise wird sichergestellt, daß die Entfernung der Phospholipide zurückgedrängt wird, die gewünschte Extraktion der Fette und Cholesterinderivate jedoch optimal verläuft.

Aufgrund der Empfindlichkeit der weiteren Inhaltsstoffe von Eiprodukten, insbesondere des Proteinanteils, wird die Extraktion vorzugsweise bei einer Extraktionstemperatur zwischen 20 und 60°C, insbesondere zwischen 30 und 50°C, durchgeführt. Aufgrund der niedrigen Extraktionsdrücke und der günstigen spezifischen Gasdurchsatzraten ist das erfindungsgemäße Verfahren besonders wirtschaftlich mit Propan/CO₂-Mischungen zwischen 90/lO und 50/50, insbesondere zwischen 80/20 und 60/40. In diesem bevorzugten Arbeitsbereich, in dem die Gasmischungen verfahrensgemäß flüssig vorliegen, kann der Extraktionsdruck 10 bis lOO bar betragen. Durch die schonenden Verfahrensparameter findet keine Denaturierung der Proteine statt. Die eingesetzte Menge an Gasmischung kann in weiten Grenzen variiert werden und richtet sich im wesentlichen nach der Menge der zu entfernenden Fette und Cholesterinderivate sowie nach der Zusammensetzung des Extraktionsgemisches: Bei Propananteilen > 70 Gew.-% reichen in der Regel 1 bis 30 kg pro kg Ausgangsmaterial aus, um eine zufriedenstellende Verringerung der gewünschten Inhaltsstoffe zu erzielen. Bei Propananteilen < 30 Gew.-% kann die notwendige Gasmenge bis auf lOO kg pro kg Ausgangsmaterial ansteigen, um das Extraktionsziel zu erreichen

Im Anschluß an die Extraktion können die im verdichteten Gasgemisch gelöst vorliegenden, extrahierten Fette und Cholesterinderivate durch Verdampfung und/oder Druckabsenkung aus dem Lösemittelgemisch wieder abgeschieden werden. Das Gasgemisch kann dann anschließend nach Verflüssigung und/oder Verdichtung wieder für die weitere Extraktion der Eiprodukte herangezogen werden, so daß man eine geringe Menge an Gasgemisch ständig im Kreis führen kann. Dadurch wird die Wirtschaftlichkeit des Verfahrens deutlich erhöht.

Es ist im Rahmen der Erfindung auch möglich, durch Variation der Extraktionsbedingungen die Fette nur teilweise aus den Eiprodukten zu extrahieren, falls dies aus bestimmten Gründen erwünscht ist. So kann durch Variation des Extraktionsdruckes, der Extraktionstemperatur sowie der Gasmengen das Verhältnis von Fetten zu Cholesterinderivaten gezielt gesteuert werden, wodurch die Effektivität des Verfahrens zusätzlich gesteigert wird.

Mit Hilfe der Erfindung ist es möglich, cholesterin- und fettarme Produkte auf Eibasis mit guten sensorischen Eigenschaften herzustellen, deren Cholesterin- und Fettgehalt um mehr als 85 % reduziert ist, wobei jedoch die Extraktion der Phospholipide zurückgedrängt ist und diese stark im Extraktionsrückstand angereichert werden.

Die nachfolgenden Beispiele sollen die Erfindung näher erläutern.

### Beispiele

Die Versuche wurden in einer Hochdruck-Extraktionsanlage für Feststoffe mit einem Autoklavenvolumen von 4 l durchgeführt. Für den Einzelversuch wurde eine Menge von 1 000 bis 1 500 g Ausgangsmaterial eingesetzt.

Das in den Beispielen 1 bis 6 untersuchte Ausgangsmaterial (Eigelbpulver) hatte folgende Zusammensetzunq im Lipidanteil:

| | |
|---|---|
| Gesamtfett (Gew.-%) | 64,3 |
| Cholesterin (Gew.-%) | 2,3 |
| Phospholipide (Gew.-%) | 17,5 |

LM bedeutet Lösemittelgemisch
AM bedeutet Ausgangsmaterial

### Beispiel 1 (Vergleichsbeispiel mit reinem Propan)

| Extraktionsmedium | Propan |
|---|---|
| Verfahrensparameter: | |
| Extraktionsdruck (bar) | 20 |
| Extraktionstemperatur (°C) | 50 |
| LM/AM (kg/kg) | 15 |

| Extrakt: | |
|---|---|
| Ausbeute (Gew.-%) | 43,6 |
| Phospholipidgehalt (Gew.-%) | 5,25 |
| Cholesteringehalt (Gew.-%) | 4,9 |

| Rückstand (Produkt): | |
|---|---|
| Ausbeute (Gew.-%) | 55,1 |
| Cholesteringehalt (Gew.-%) | 0,14 |
| Gesamtfett (Gew.-%) | 33,5 |
| Phospholipide im Gesamtfett (Gew.-%) | 81 |
| Phospholipide im Rückstand, abs. (Gew.-%) | 27,1 |

### Beispiel 2 (Vergleichsbeispiel mit reinem CO₂)

| Extraktionsmedium | CO₂ |
|---|---|
| Verfahrensparameter: | |
| Extraktionsdruck (bar) | 300 |
| Extraktionstemperatur (°C) | 50 |
| LM/AM (kg/kg) | 130 |

| Extrakt: | |
|---|---|
| Ausbeute (Gew.-%) | 38,5 |
| Phospholipidgehalt (Gew.-%) | 0,1 |
| Cholesteringehalt (Gew.-%) | 5,9 |

| Rückstand (Produkt): | |
|---|---|
| Ausbeute (Gew.-%) | 58,4 |
| Cholesteringehalt (Gew.-%) | 0,15 |
| Gesamtfett (Gew.-%) | 40 |
| Phospholipide im Gesamtfett (Gew.-%) | 75 |
| Phospholipide im Rückstand, abs. (Gew.-%) | 30 |

### Beispiel 3

| Extraktionsmedium | Propan/Kohlendioxid |
|---|---|
| Mischungsverhältnis | 90/10 |

| Verfahrensparameter: | |
|---|---|
| Extraktionsdruck (bar) | 30 |
| Extraktionstemperatur (°C) | 50 |
| LM/AM (kg/kg) | 15 |

| Extrakt: | |
|---|---|
| Ausbeute (Gew.-%) | 41,6 |
| Phospholipidgehalt (Gew.-%) | 3,75 |
| Cholesteringehalt (Gew.-%) | 5,4 |

| Rückstand (Produkt): | |
|---|---|
| Ausbeute (Gew.-%) | 56,3 |
| Cholesteringehalt (Gew.-%) | 0,14 |
| Gesamtfett (Gew.-%) | 36,3 |
| Phospholipide im Gesamtfett (Gew.-%) | 76 |
| Phospholipide im Rückstand, abs. (Gew.-%) | 27,6 |

### Beispiel 4

| Extraktionsmedium | Propan/Kohlendioxid |
|---|---|
| Mischungsverhältnis | 80/20 |

| Verfahrensparameter: | |
|---|---|
| Extraktionsdruck (bar) | 40 |
| Extraktionstemperatur (°C) | 50 |
| LM/AM (kg/kg) | 15 |

| Extrakt: | |
|---|---|
| Ausbeute (Gew.-%) | 44,3 |
| Phospholipidgehalt (Gew.-%) | 1 |
| Cholesteringehalt (Gew.-%) | 5,6 |

| Rückstand (Produkt): | |
|---|---|
| Ausbeute (Gew.-%) | 57,7 |
| Cholesteringehalt (Gew.-%) | 0,18 |
| Gesamtfett (Gew.-%) | 38,0 |
| Phospholipide im Gesamtfett (Gew.-%) | 77 |
| Phospholipide im Rückstand, abs. (Gew.-%) | 29,3 |

### Beispiel 5

| Extraktionsmedium | Propan/Kohlendioxid |
|---|---|
| Mischungsverhältnis | 70/30 |

| Verfahrensparameter: | |
|---|---|
| Extraktionsdruck (bar) | 50 |
| Extraktionstemperatur (°C) | 45 |
| LM/AM (kg/kg) | 30 |

| Extrakt: | |
|---|---|
| Ausbeute (Gew.-%) | 38,55 |
| Phospholipidgehalt (Gew.-%) | 0,2 |
| Cholesteringehalt (Gew.-%) | 5,9 |

| Rückstand (Produkt): | |
|---|---|
| Ausbeute (Gew.-%) | 58,4 |
| Cholesteringehalt (Gew.-%) | 0,16 |
| Gesamtfett (Gew.-%) | 39,9 |
| Phospholipide im Gesamtfett (Gew.-%) | 75 |
| Phospholipide im Rückstand, abs. (Gew.-%) | 29,9 |

### Beispiel 6

| Extraktionsmedium | Propan/Kohlendioxid |
|---|---|
| Mischungsverhältnis | 30/70 |

| Verfahrensparameter: | |
|---|---|
| Extraktionsdruck (bar) | 250 |
| Extraktionstemperatur (°C) | 45 |
| LM/AM (kg/kg) | 75 |

| Extrakt: | |
|---|---|
| Ausbeute (Gew.-%) | 38,5 |
| Phospholipidgehalt (Gew.-%) | 0,15 |
| Cholesteringehalt (Gew.-%) | 5,8 |

| Rückstand (Produkt): | |
|---|---|
| Ausbeute (Gew.-%) | 58,2 |
| Cholesteringehalt (Gew.-%) | 0,18 |
| Gesamtfett (Gew.-%) | 39,5 |
| Phospholipide im Gesamtfett (Gew.-%) | 74 |
| Phospholipide im Rückstand, abs. (Gew.-%) | 29,6 |

### Beispiel 7

Das in Beispiel 7 untersuchte Ausgangsmaterial (Volleipulver) hatte im Lipidanteil folgende Zusammensetzung:

| | |
|---|---|
| Gesamtfett (Gew.-%) | 41,2 |
| Cholesterin (Gew.-%) | 1,4 |
| Phospholipide (Gew.-%) | 13,9 |

| Extraktionsmedium | Propan/Kohlendioxid |
|---|---|
| Mischungsverhältnis | 70/30 |

| Verfahrensparameter: | |
|---|---|
| Extraktionsdruck (bar) | 50 |
| Extraktionstemperatur (°C) | 45 |
| LM/AM (kg/kg) | 25 |

| Extrakt: | |
|---|---|
| Ausbeute (Gew.-%) | 25,8 |
| Phospholipidgehalt (Gew.-%) | 0,2 |
| Cholesteringehalt (Gew.-%) | 1,9 |

| Rückstand (Produkt): | |
|---|---|
| Ausbeute (Gew.-%) | 73,8 |
| Cholesteringehalt (Gew.-%) | 0,12 |
| Gesamtfett (Gew.-%) | 21,4 |
| Phospholipide im Gesamtfett (Gew.-%) | 85 |
| Phospholipide im Rückstand, abs. (Gew.-%) | 18,2 |

## Patentansprüche

1. Verfahren zur Herstellung von fett- und cholesterinreduzierten pulverförmigen Produkten auf Eibasis mit einem hohen Gehalt an Phospholipiden durch Extraktion mit verdichteten Gasen,
dadurch gekennzeichnet,
daß man das pulverförmige Ausgangsmaterial mit einem Lösemittelgemisch bestehend aus Propan und C0₂ in einem Mischungsverhältnis zwischen 95/5 und 5/95 Gew.-% bei einem Druck < 300 bar und einer Temperatur < 70°C extrahiert.

2. Verfahren nach Anspruch 1,
dadurch gekennzeichnet,
daß das Mischungsverhältnis von Propan zu C0₂ zwischen 90/10 und 50/50 Gew.-% liegt.

3. Verfahren nach den Ansprüchen 1 und 2,
dadurch gekennzeichnet,
daß das Mischungsverhältnis von Propan und C0₂ zwischen 80/20 und 60/40 Gew.-% liegt.

4. Verfahren nach den Ansprüchen 1 bis 3,
dadurch gekennzeichnet,
daß die Extraktionstemperatur 20 bis 60°C, insbesondere 30 bis 50°C, beträgt.

5. Verfahren nach den Ansprüchen 1 bis 4,
dadurch gekennzeichnet,
daß man die Extraktion bei einem Druck von 10 bis 100 bar durchführt.

6. Verfahren nach den Ansprüchen 1 bis 5,
dadurch gekennzeichnet,
daß man pro kg Ausgangsmaterial 1 bis 100 kg des Lösemittelgemisches verwendet.

7. Verfahren nach den Ansprüchen 1 bis 6,
dadurch gekennzeichnet,
daß man die Fette und Cholesterinderivate durch Verdampfung und/oder Druckabsenkung aus dem Lösemittelgemisch abscheidet.

## Claims

1. Process for the production of fat-reduced and cholesterol-reduced powdered egg-based products with a high content of phospholipids by extraction with compressed gases,
wherein
the powdered starting material is extracted with a solvent mixture composed of propane and CO₂ in a mixing ratio between 95/5 and 5/95 % by weight at a pressure of < 300 bar and a temperature of < 70°C.

2. Process as claimed in claim 1,
wherein
the mixing ratio of propane and CO₂ is between 90/10 and 50/50 % by weight.

3. Process as claimed in claims 1 and 2,
wherein
the mixing ratio of propane and CO₂ is between 80/20 and 60/40 % by weight.

4. Process as claimed in claims 1 to 3,
wherein
the extraction temperature is 20 to 60°C, in particular 30 to 50°C.

5. Process as claimed in claims 1 to 4,
wherein
the extraction is carried out at a pressure of 10 to 100 bar.

6. Process as claimed in claims 1 to 5,
wherein
1 to 100 kg of the solvent mixture is used per kg starting material.

7. Process as claimed in claims 1 to 6,
wherein
the fats and cholesterol derivatives are separated from the solvent mixture by evaporation and/or pressure reduction.

## Revendications

1. Procedé de préparation de produits en poudre à base d'oeufs, à teneur réduite en graisses et en cholestérol et à haute teneur en phospholipides, par extraction avec des gaz comprimés, caractérisé en ce que l'on extrait le produit de départ en poudre avec un mélange de solvants constitué de propane et de CO₂ en un rapport de mélange compris entre 95/5 et 5/95 % en masse, sous une pression inférieure à 300 bar et à une température inférieure à 70°C.

2. Procédé selon la revendication 1, caractérisé en ce que le rapport de mélange du propane au CO₂ est compris entre 90/10 et 50/50 % en masse.

3. Procédé selon les revendications 1 et 2, caractérisé en ce que le rapport de mélange du propane et du CO₂ est compris entre 80/20 et 60/40 % en masse.

4. Procédé selon les revendications 1 à 3, caractérisé en ce que la température d'extraction est de 20 à 60°C, en particulier de 30 à 50°C.

5. Procédé selon les revendications 1 à 4, caractérisé en ce que l'on effectue l'extraction sous une pression de 10 à 100 bar.

6. Procédé selon les revendications 1 à 5, caractérisé en ce que l'on utilise 1 à 100 kg de mélange de solvant par kg de produit de départ.

7. Procédé selon les revendications 1 à 6, caractérisé en ce que l'on sépare les graisses et les dérivés de cholestérol du mélange de solvants par évaporation et/ou réduction de la pression.
